# EUROPÄISCHE PATENTSCHRIFT

(11) **EP 1 722 027 B1**
(45) Veröffentlichungstag und Bekanntmachung des Hinweises auf die Patenterteilung: **15.08.2012**
(21) Anmeldenummer: 06009430.7
(22) Anmeldetag: 08.05.2006
(51) Int. Cl.: D06F 58/10, A61L 2/06, A61L 2/14, A61L 2/24

(54) **Vorrichtung zur Reinigung und Entkeimung von Matratzen**
Device for cleaning and sterilisation of matresses
Dispositif pour nettoyer et stériliser des matelas

(30) Priorität: 10.05.2005 DE 202005007406 U
(43) Veröffentlichungstag der Anmeldung: 15.11.2006
(73) Patentinhaber: Schöttle, Günther, 72202 Nagold (DE)
(72) Erfinder: Schöttle, Günther, 72202 Nagold (DE)
(74) Vertreter: Peterreins, Frank

(56) Entgegenhaltungen:
- EP-A- 0 148 385
- DE-A1- 2 708 068
- DE-A1- 3 047 938
- DE-A1- 4 133 872
- US-A- 3 482 931
- US-A- 4 549 362
- PATENT ABSTRACTS OF JAPAN Bd. 2003, Nr. 06, 3. Juni 2003 (2003-06-03) & JP 2003 038897 A (TANABE KOGYO KK), 12. Februar 2003 (2003-02-12)

## Beschreibung

Die vorliegende Erfindung betrifft eine Vorrichtung zur Reinigung, Trocknung, Entkeimung und Geruchsneutralisierung von einer oder mehreren Matratzen, insbesondere für Betten in Hotels, Alten-/Pflegeheimen und Krankenhäusern.

Im Stand der Technik sind Verfahren zur Desinfektion, zur Reinigung und zum Waschen von Matratzen bekannt. Ein besonderer Bedarf für solche Verfahren besteht insbesondere bei Betten in Hotels, Alten-/Pflegeheimen und Krankenhäusern. Matratzen werden hier besonders stark belastet, z.B. durch menschliche Ausscheidungen. Die meisten bekannten Reinigungsverfahren beruhen auf einer O-berflächenreinigung durch thermische Desinfektion, chemische Reinigung, Infrarot-Strahlung oder UV-Strahlung. Eine vorwiegend nur oberflächennahe Reinigung hat den Nachteil, dass hygienische Probleme im Inneren der Matratze nicht oder nur unzulänglich gelöst werden. Viele der bekannten Verfahren haben zudem den Nachteil, dass die Matratzen bei der Reinigung in Mitleidenschaft gezogen werden. So kann starke Erhitzung zu Faserbruch, Verlust von Spannkraft oder Zerstörung von Schaumstoffen führen. Ein weiterer Nachteil der thermischen Desinfektion ist der hohe anlagentechnische Aufwand. Bleichmittel verursachen meist ein unerwünschtes Ausbleichen der Oberflächenstruktur. Chemische Reinigungsverfahren belasten zumeist die Umwelt und sind ebenfalls kostenintensiv.

Folge dieser Schwierigkeiten beim Reinigen von Matratzen ist häufig die Verwendung von wasserundurchlässigen Schichten zum Schutz der Matratzen. Dies mindert zumeist das Wohlbefinden der Nutzer der Matratzen und kann darüber hinaus negative gesundheitliche Folgen durch eine zu geringe effektive Atmungsfähigkeit der Matratze nach sich ziehen, wie z.B. Hauterkrankungen oder Wundliegen.

DE 30 47 938 A1 offenbart ein Verfahren zum Sterilisieren von Matratzen, Wäschesäcken u.dgl. Waren, bei dem an eine Sterilisierkammer eines Sterilisiergerätes eine mit der Druckseite und mit der Saugseite eines eingebauten Belüfters verbundene Ringleitung angeschlossen ist. Zur Durchführung dieses Verfahrens ist ein entsprechendes Sterilisiergerät weiterhin mit einer an den ansaugseitigen Abschnitt der Ringleitung angeschlossenen Zuleitung für ein Sterilisationsgas und mit einer an deren druckseitigen Abschnitt angeschlossenen, der Entfernung des Sterilisationsgases aus der Sterilisierkammer dienenden Ableitung versehen.

DE 27 08 068 A offenbart ein Verfahren und ein dazugehöriges Gerät zum zwangsweisen Desinfizieren, Desodorieren, Entlüften, Durchlüften, Trocknen, Entstauben und Entmotten, speziell von Matratzen oder anderen Gegenständen.

US 3 482 931 A offenbart eine Vorrichtung derart ausgebildet, dass ein Raum innerhalb der Vorrichtung, in dem das Reinigen und Desinfizieren erfolgt, mittels einer Auflage für die Bettwaren in zwei Kammern aufgeteilt ist, und dass eine der Kammern mit der Saugseite eines Gebläses und die andere Kammer mit der die Vorrichtung umgebenden Außenluft in Verbindung steht, und diese Verbindungen mittels einer Weiche wahlweise vertauschbar sind, und dass Weichen vorhanden sind, die eine Umwälzung der Luft in der Vorrichtung zwischen den beiden Kammern ermöglichen.

JP 2003 038897 A offenbart eine Futon Trockner, der eine Vielzahl von Futons und Matratzen in kurzer Zeit trocknen und sterilisieren kann. Er besteht aus einer Trockenkammer, mehreren Regalebenen, einem Gebläse, einem Lufterwärmer, einem Einlass und einem Auslass für heiße Luft und einem Befestigungsbereich mit Belüftungslöchern.

DE 44 26 647 A1 zeigt Verfahren und Vorrichtungen die zum Behandeln von textilen Haushaltsgegenstänolen zum zwecke der Bekämpfung von Ungeziefer, wobei Begasung in Form ozonhaltiger luft oder Ozun haltigen sauerstoffs erfolgt. Als Hanshattsgegenstänole werden auch Matiatzen erwähnt.

JP 2001 16 17 97 A zeigt eine Ozon-und Ionenerzenbgungseinrichtung zur Entkeimung von Gegenständen, wie auch Matratzen.

Es ist daher die Aufgabe der vorliegenden Erfindung, eine Vorrichtung zum Reinigen und Entkeimen von Matratzen bereitzustellen, die eine gründliche und gleichzeitig schonende Reinigung und Entkeimung ermöglicht. Außerdem sollte eine Reinigung mit geringen Betriebskosten verbunden sein.

Die Aufgabe wird durch eine Vorrichtung zum Reinigen und Entkeimen einer oder mehrerer Matratzen gemäß Anspruch 1 gelöst. Die abhängigen Ansprüche enthalten vorteilhafte Ausgestaltungen der Erfindung. Nachfolgenol werden Aspelde der Erfinding unter Mitausrichtung auf bevorzugte Ausführungsbeispiele hervorgehoren. Die Vorrichtung nach der vorliegenden Erfindung weist eine Matratzenkammer mit einer Abteilung oder mehreren Abteilungen zur Aufnahme jeweils einer Matratze auf. Es ist mindestens eine Zuluftöffnung und mindestens eine Abluftöffnung an der Matratzenkammer vorgesehen, wobei die Zu- und Abluftöffnungen derart in der Matratzenkammer angeordnet sind, dass zuströmende Luft von der mindestens einen Zuluftöffnung durch eine Matratze oder durch mehrere Matzratzen hindurchströmen und über die mindestens eine Abluftöffnung abströmen kann. Die Luft wird dabei vorzugsweise über einen Zuluftventilator an die Matratzenkammer zugeführt. Um eine möglichst homogene Verteilung der Luftströmung über die Matratze zu erreichen, kann für eine Abteilung jeweils ein Lochblech als Matratzenauflage mit einer homogenen Verteilung der Löcher vorgesehen sein. Gemäß der Erfindung können mehrere Abteilungen in einer Matratzenkammer vorgesehen werden. Hierdurch können mehrere Matratzen gleichzeitig gereinigt werden, was zu einer deutlichen Senkung der Reinigungskosten pro Matratze führt.

Die Vorrichtung nach der vorliegenden Erfindung ermöglicht eine geeignete Einbringung bzw. Lagerung der Matratze, so dass optimale Bedingungen für deren Reinigung, Trocknung und Entkeimung gegeben sind. Jede eingelegte Matratze wird gleichmäßig über ihre gesamte Querschnittsfläche von Luft durchströmt. Hierzu wird Luft vorzugsweise auf einer Seite eingeblasen und auf der anderen Seite durch ein zweites Gebläse abgesaugt. Die Durchströmung der gesamten Matratze ist von besonderer Bedeutung, da erst sie eine schnelle und tiefgehende Reinigung der Matratze ermöglicht.

Um zu gewährleisten, dass möglichst die gesamte zugeführte Luft durch die Matratze geströmt wird, sind die Seitenwände der Abteilungen vorzugsweise angepasst, die Matzratze an den Seiten anzudrücken. Hierzu kann mindestens eine Seitenwand der mindestens einen Abteilung verstellbar ausgeführt sein, um eine aufgenommene Matratze seitlich anzudrücken, insbesondere an einer Längsseite und/oder an einer Kopf-/Fußseite.

Im Bereich einer Klappe zum Einbringen einer Matratze in eine Abteilung können eine oder mehrere Bürsten zur Reinigung der Matratzenoberfläche vorgesehen sein, so dass die Matratze während des Einbringens in eine Abteilung abgebürstet wird. Dabei ist es vorteilhaft, im Bereich der Klappe eine Einrichtung zur Absaugung der Matratzenoberfläche vorzusehen, um eine Matratze während des Einbringens in eine Abteilung über einen Spalt abzusaugen. Zur Absaugung der Partikel ist der Anschluss eines Hochleistungsgebläses an die Spaltabsaugung vorgesehen. Dieses Gebläse kann mit einem als Patronenfilter zur Abscheidung von Staub-, Flusen, Hautpartikeln und Milben ausgerüstet werden.

Luft, die von außerhalb der Vorrichtung angesaugt wird, wird durch einen Filter gereinigt. Die Luft kann nach einer besonders vorteilhaften Ausgestaltung der Erfindung ionisiert werden bevor sie zur Matratzenkammer geleitet wird; insbesondere können Sauerstoffmoleküle aufgespalten werden. Dabei handelt es sich um eine natürliche Art Gerüche abzubauen und Keime zu inaktivieren bzw. abzutöten. Dieser Naturprozess kann durch elektrisch betriebene Ionisationsgeräte kopiert werden. Nach dem Prinzip der Koronaentladung werden Sauerstoffmoleküle in Sauerstoffradikale gespalten. Diese äußerst reaktiven Atome streben nach einer Verbindung mit anderen Stoffen, die ein Elektronendefizit haben. Durch die Anlagerung von Sauerstoffradikalen werden alle erreichbaren organischen und teilweise anorganischen Gase unmittelbar oxidiert und damit nicht mehr umkehrbar geruchlich neutralisiert. Der gleiche Prozess vollzieht sich prinzipiell auch bei Mikroorganismen. Die technische Realisierung erfolgt durch Hochspannungsionisationsröhren, die in Form von Glasröhren angeordnet sind.

Die Ionisierte Luft dient somit der Reinigung und Entkeimung. Zur besonders gründlichen Reinigung kann die Vorrichtung im Umluftbetrieb betrieben werden, hier kann die Luft z.B. 100-fachen umgewälzt werden. Dieses strahlungsfreie und chemiefreie Verfahren, welches aus der Lebensmitteltechnologie bekannt ist, erzielt Keimabtötungsraten von bis zu 99%. Diese Art der Reinigung ist besonders schonend, so dass eine Matratze häufig gereinigt werden kann. Es ist eine Luftführung vorgesehen, so dass die Einrichtung zum Ionisieren umgangen werden kann. Diese Bypassführung kann zum Schutz der Ionisationsvorrichtung vor sehr hohen Temperaturen verwendet werden. Alternativ kann die Luft auch mit chemischen Stoffen zur Reinigung, beispielsweise mit Desinfektionsmittel versetzt werden. Die Luft kann ebenfalls mit Aromastoffen versetzt werden, um ein gewünschtes Schlafklima oder Raumgeruch zu erzielen. Darüber hinaus besteht die Möglichkeit, die Luft zu erhitzen. Nach der gewünschten Konditionierung der Luft wird diese über einen Zuluftventilator in die Matratzenkammer eingeblasen.

Zwischen Zuluftventilator und Matratzenkammer kann nach einer besonders vorteilhaften Ausgestaltung der Erfindung ein Pulsator geschaltet werden. Dieser erzeugt einen ungleichmäßigen, impulsartigen Luftstrom, um durch Druckwellen Schadstoffe in den Matratzen aufzulockern und anschließend abzutransportieren.

Auf der Abluftseite wird die Luft durch ein Gebläse aus der Matratzenkammer gesaugt. Die abgesaugt Luft kann durch einen hierfür vorgesehenen Filter gereinigt werden. Dieser Filter kann jedoch auch durch eine geeignete Bypassleitung umgangen werden, um ihn z.B. vor Beschädigung bei einem Betrieb mit sehr hoher Temperatur zu schützen. Die Bypassleitung ist insbesondere für den Umluftbetrieb bei thermischer Keimabtötung (ca. 105° C bis 120°C) von Bedeutung. Vom Gebläse des Abluftbereichs aus kann die Luft nach außen abgeblasen werden, oder innerhalb der Vorrichtung zum Zuluftbereich zurückgeführt werden. Die Luft kann in einem geschlossenen Luftkreislauf geführt werden.

Durch eine Umschalt- und/oder Steuereinrichtung zur gestuften oder stufenlosen Umschaltung und/oder Steuerung kann zwischen Luftumwälzung in der Vorrichtung und Ansaugung von Frischluft und Abblassung von Brauchluft gewählt werden, wobei insbesondere auch eine Kombination von Ansaugung von Frischluft mit dem Abblasen von Brauchluft eingestellt werden kann. Hierzu dienen Klappen in der Vorrichtung zur Reinigung und/oder Entkeimung von Matratzen. Zusätzlich sind Luftführungsklappen an den einzelnen Abteilungen vorgesehen, um die Luftzuführung bzw. Absaugung für jede Abteilung einzeln kontrollieren zu können. Die Umschalt- und/oder Steuereinrichtung ermöglicht die Programmierung und die Kontrolle von bzw. über eine beliebige Abfolge von Betriebsschritten und gewünschter Prozessparameter, z.B. Temperatur, Dauer, Strömungsgeschwindigkeit. Die Messung der Konzentration organischer Schadstoffe ist ebenfalls möglich. Diese Messung kann auch während des Betriebs durchgeführt werden, ihr Ergebnis kann über eine Anzeige verfügbar gemacht werden und/oder als Regelgröße innerhalb der Umschalt- und/oder Steuereinrichtung verwendet werden.

Die erfindungsgemäße Vorrichtung zum Reinigen und Entkeimen einer oder mehrerer Matratzen kann sowohl stationär, als auch mobil, z.B. auf einen Lastkraftwagen montiert, eingesetzt werden.

Die vorliegende Erfindung wird nachfolgend anhand bevorzugter Ausführungsbeispiele unter Bezug auf die beigefügten Zeichnungen erläutert.
- Fig. 1: zeigt eine Abteilung einer Matratzenkammer eingebettet in eine Vorrichtung zur Reinigung und Entkeimung von Matratzen nach einer Ausführungsform der vorliegenden Erfindung,
- Fig. 2: zeigt den inneren Aufbau der Vorrichtung zur Reinigung und Entkeimung von Matratzen nach einer Ausführungsform der vorliegenden Erfindung,
- Fig. 3: zeigt die Steuereinheit und die Stellmotoren der Vorrichtung zur Reinigung und Entkeimung von Matratzen nach einer Ausführungsform der vorliegenden Erfindung, und
- Fig. 4: zeigt schematisch die Luftführung innerhalb der Vorrichtung zur Reinigung und Entkeimung von Matratzen nach einer Ausführungsform der vorliegenden Erfindung.

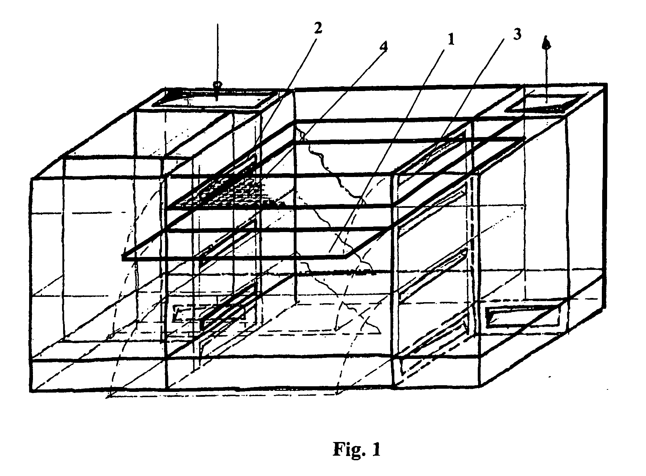

Fig. 1 zeigt eine Matratzenkammer mit drei Abteilungen für je eine Matratze gemäß einem Ausführungsbeispiel der vorliegenden Erfindung. In dieser Darstellung ist jede Abteilung mit einer Klappe 1 versehen, durch die eine Matratze in die Abteilung gelegt bzw. entfernt werden kann. Zudem ist ein Zwischenboden, vorzugsweise als Lochblech 4 ausgeführt, dargestellt. Weiterhin sind eine Zuluft-öffnung 2 unterhalb des Zwischenbodens und eine Abluftöffnung 3 oberhalb der einzulegenden Matratze zu sehen. Diese Anordnung stellt damit sicher, dass jede Matratze sehr gleichmäßig und über ihre gesamte Fläche von Luft durchströmt wird.

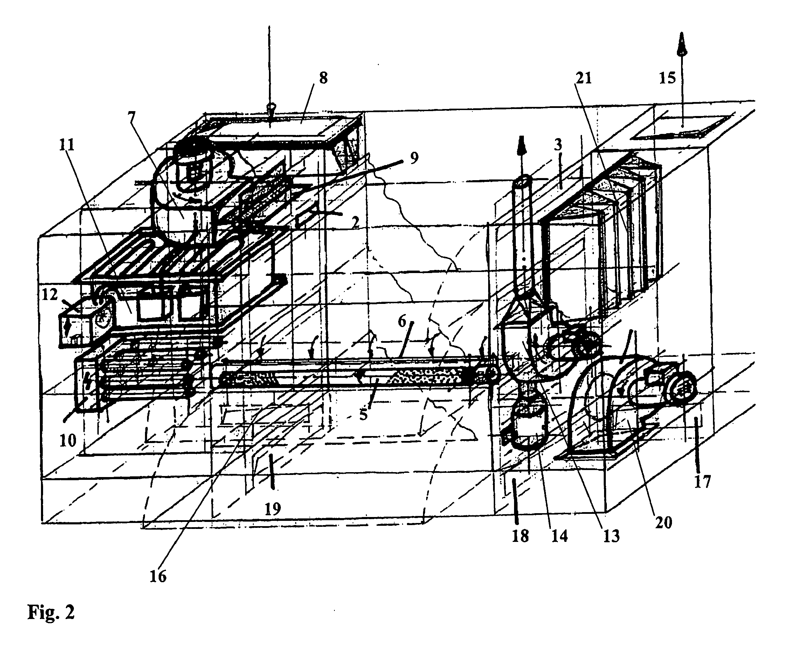

Fig. 2 zeigt den inneren Aufbau der Vorrichtung zur Reinigung und Entkeimung von Matratzen nach einem Ausführungsbeispiel der vorliegenden Erfindung. Beim Einschieben einer Matratze in eine Abteilung wird diese oberflächlich durch eine Bürste 5 und eine Spaltabsaugung 6 gereinigt. An die Spaltluftabsaugung 6 ist ein Hochleistungsgebläse zur Absaugung 13 angeschlossen, welches mit einem Patronenfilter 14 zur Abscheidung von Staub-, Flusen-, Hautpartikeln und Milben versehen ist.

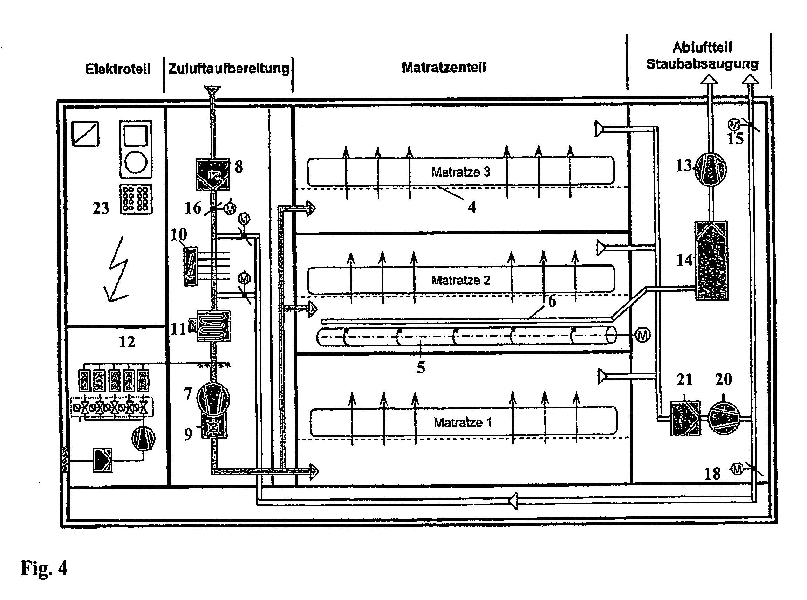

Die Vorrichtung zur Reinigung und Entkeimung von Matratzen kann sowohl im Frischluftbetrieb als auch im Umluftbetrieb betrieben werden, hierauf wird im Zusammenhang mit Fig. 4 ausführlich eingegangen. An dieser Stelle wird das Hauptaugenmerk auf die Funktion der einzelnen Elemente und deren Zusammenwirken gelegt. Auf der linken Seite der Abbildung befindet sich die Luftzuführung, wobei die angesaugte Außenluft durch einen Filter 8 gereinigt wird. Danach passiert die frische Luft die Außenluftklappe 16 und gelangt so in den Hauptbereich der Zuluftseite. Hier kann sie durch ein Elektroregister 11 erhitzet werden, um so zur Matratzentrocknung eingesetzt zu werden. Wahlweise bzw. zusätzlich kann sie durch ein Ionisationsgerät 10 geleitet werden, um zur Desinfektion der Matratzen eingesetzt zu werden. Weiterhin besteht die Möglichkeit, die Zuluft durch eine Aromapumpe mit Aromacontainern 12 mit Aromastoffen zu versetzen.

Die nach Wunsch behandelte Luft kann über einen Zuluftventilator 7 und einen Pulsator 9, der einen ungleichmäßigen impulsartigen Luftstrom erzeugt, weitergeleitet werden. Durch diesen impulsartigen Luftstrom wird der Reinigungsprozess beschleunigt und Verhärtungen in der Matratze können aufgelockert werden. Die so konditionierte Luft wird durch die Zulassöffnungen 2 in die Abteilungen der Matratzenkammer eingeleitet. Hier durchströmt sie wie oben erläutert die gesamte Matratze. Die Luft wird durch ein Absaug- bzw. Umluftgebläse 20 durch die Abluftöffnungen 3 abgesaugt. Auf diesem Weg wird die Luft durch einen Taschenfilter 21 von luftgetragenen Partikel befreit. Es ist ebenfalls eine Luftführung vorgesehen, die den Taschenfilter 21 umgeht, um ihn beim Betreib mit sehr hoher Temperatur vor Beschädigung zu schützen. Je nach Wahl des Betriebsmodus wird die gereinigte Luft entweder über die Fortluftklappe 17 und den Fortluftaustritt 15 nach außen abgeblasen, oder sie wird über die abluftseitige Umluftklappe 18 und die zuluftseitige Umluftklappe 19 wieder in die Zuluftseite der Vorrichtung eingespeist.

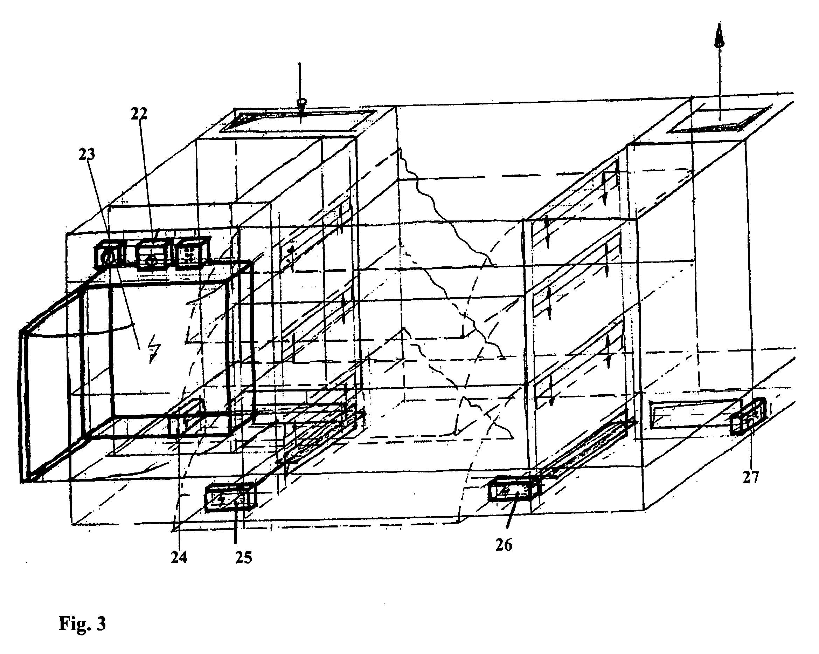

Fig. 3 zeigt die Steuereinheit und die Stellmotoren der Vorrichtung zur Reinigung und Entkeimung von Matratzen nach der vorliegenden Erfindung. Die Stellmotoren 24, 25, 26, 27 ermöglichen eine automatische Ansteuerung der Klappen 16, 19, 18, 17. Hierdurch ist es möglich mittels des Befehlsgeräts mit Anzeigedisplay 22, eine gewünschte Reihenfolge von Prozessschritten zu programmieren. Die gewünschte Prozessführung wird mit Hilfe des Elektroschranks mit Schalt- und Funktionsteilen 23 umgesetzt, dieser steuert ebenfalls die Stellmotoren 24, 25, 26, 27.

Fig. 4 zeigt schematisch die Luftführung innerhalb der Vorrichtung zur Reinigung und Entkeimung von Matratzen nach der vorliegenden Erfindung. Die Vorrichtung kann wahlweise Luft von außen ansaugen bzw. nach außen abblasen oder Luft innerhalb der Vorrichtung umwälzen. Der Umwälzbetrieb ist von besonderer Bedeutung für die Matratzentrocknung mittels erwärmter Luft und für die Entkeimung mittels ionisierter Luft. Der Frischluftbetrieb kann z.B. für das einfache Lüften von Matratzen verwendet werden.

Im Frischluftbetrieb wird die Außenluft über den Filter 8 und die Außenluftklappe 16 angesaugt. Die Luft kann im Zuluftaufbereitungsbereich erhitzt 11, ionisiert 10 und aromatisiert 12 werden. Anschließend wird sie über den Zuluftventilator 7 und den Pulsator 9 in die Matratzenkammer geleitet. Hier durchströmt sie von unten die Lochblechelemente 4 und gleichmäßig die eingelegten Matratzen. Die abgesaugte Luft wird im Abluftteil durch den Filter 21 von luftgetragenen Partikeln befreit. Die Luft kann alternativ am Filter vorbeigeführt werden, ohne mit diesem in Kontakt zu kommen. Von hieraus kann die Luft durch das Absauge bzw. Umluftgebläse 20 entweder über eine Fortluftöffnung 15 nach außen abgeleitet werden, oder sie kann unterhalb der Matratzenkammem über eine abluftseitige Umluftklappe 18 und einen Hohlraum wieder dem Luftzubereitungsbereich zugeführt werden.

Die Luftführung der Spaltabsaugung 6 ist von der bisher erläuterten Luftführung unabhängig. Die Luft, die von der Spaltabsaugung 6 abgesaugt wird, wird durch den Filter 14 über das Hochleistungsgebläse 13 nach außen abgegeben.

### Bezeichnungsliste:

- 1: Klappe zum Einlegen einer Matratze
- 2: Zuluftöffnung
- 3: Abluftöffnung
- 4: Lochblech
- 5: Bürste zur Reinigung der Matratzenoberfläche
- 6: Spaltabsaugung zur Absaugung der Matratzenoberfläche
- 7: Zuluftventilator
- 8: Filter für Außenluft
- 9: Pulsator, zur Erzielung eines ungleichmäßigen, impulsartigen Luftstroms
- 10: Ionisationsgerät zur Entkeimung der Luft
- 11: Elektroheizregister zur Erwärmung der Luft
- 12: Aromapumpe mit Aromacontainer
- 13: Hochleistungsgebläse zur Absaugung
- 14: Patronenfilter zum Abscheiden von Staub-/Flusen-/Milben und Hautpartikeln
- 15: Fortluftaustritt
- 16: Außenluftklappe
- 17: Fortluftklappe
- 18: Umluftklappe, abluftseitig
- 19: Umluftklappe, zuluftseitig
- 20: Absauge bzw. Umluftgebläse
- 21: Taschenfilter zur Abscheidung von Flusen und luftgetragenen Partikeln
- 22: Befehlsgerät mit Anzeigedisplay
- 23: Elektroschaltschrank mit Schalt- und Funktionsteilen
- 24: Stellmotor für Außenluft
- 25: Stellmotor für Umluftklappe, zuluftseitig
- 26: Stellmotor für Umluftklappe, fortluftseitig
- 27: Stellmotor für Fortluft

## Patentansprüche

1. Vorrichtung zum Reinigen und zum Entkeimen von einer oder mehreren Matratzen, wobei
die Vorrichtung eine Matratzenkammer mit einer Abteilung oder mehreren Abteilungen zur Aufnahme jeweils einer Matzratze aufweist, und dass mindestens eine Zuluftöffnung (2) und mindestens eine Abluftöffnung (3) an der Matratzenkammer vorgesehen sind,
wobei die Zu- und Abluftöffnungen (2, 3) derart in der Matratzenkammer angeordnet sind, dass zuströmende Luft oder dergleichen von der mindestens einen Zuluft-öffnung (2) durch eine Matratze oder durch mehrere Matzratzen hindurchströmen und über die mindestens eine Abluftöffnung (3) abströmen kann,
**dadurch gekennzeichnet, dass** für das Reinigen mindestens eine Bürste (5) zur Reinigung der Matratzenoberfläche vorgesehen ist, um die Matratze während des Einbringens in eine Abteilung abzubürsten,
und/oder eine Einrichtung (6) zur Absaugung der Matratzenoberfläche vorgesehen ist, um die Matratze während des Einbringens in die Abteilung abzusaugen, und
dass für das Entkeimen eine Einrichtung (10) zur Ionisierung von Luft, insbesondere von Sauerstoffdioxid, in Strömungsrichtung der Luft vor der Matratzenkammer vorgesehen ist, und gegebenenfalls eine Einrichtung zum Versetzen der Luft mit einem Desinfektionsmittel in Strömungsrichtung der Luft vor der Matratzenkammer vorgesehen ist.

2. Vorrichtung gemäß Anspruch 1, **dadurch gekennzeichnet, dass** Anpressprofile an den Seiten der mindestens einen Abteilung vorgesehen sind, um eine aufgenommene Matratze seitlich anzudrücken, wobei die Anpressprofile vorzugsweise verstellbar ausgebildet sind

3. Vorrichtung gemäß einem der vorhergehenden Ansprüche, **dadurch gekennzeichnet, dass** für eine Abteilung jeweils ein Lochblech (4) als Matratzenauflage vorgesehen ist, welches insbesondere eine homogene Verteilung der Löcher aufweist.

4. Vorrichtung gemäß einem der vorhergehenden Ansprüche, **dadurch gekennzeichnet, dass** eine Einrichtung (9) zur Erzeugung eines ungleichmäßigen, impulsartigen Luftstroms in Strömungsrichtung der Luft vor der Matratzenkammer vorgesehen ist.

5. Vorrichtung gemäß einem der vorhergehenden Ansprüche, **dadurch gekennzeichnet, dass** eine Einrichtung zum Aufheizen der Luft in Strömungsrichtung der Luft vor der Matratzenkammer vorgesehen ist.

6. Vorrichtung gemäß einem der vorhergehenden Ansprüche, **dadurch gekennzeichnet, dass** die Luft in einem Kreislauf in der Vorrichtung umgewälzt werden kann.

7. Vorrichtung gemäß Anspruch 6, **dadurch gekennzeichnet, dass** eine Umschalt- und/oder Steuereinrichtung zur gestuften oder stufenlosen Umschaltung und/oder Steuerung zwischen Luftumwälzung in der Vorrichtung und Ansaugung von Frischluft und Abblassung von Brauchluft vorgesehen ist, wobei insbesondere auch eine Kombination von Ansaugung von Frischluft mit dem Abblasen von Brauchluft eingestellt werden kann.

8. Vorrichtung gemäß einem der vorhergehenden Ansprüche, **dadurch gekennzeichnet, dass** eine Einrichtung zur Messung der Konzentration organischer Schadstoffe vorgesehen ist, wobei die Messung insbesondere über eine Anzeige dargestellt werden kann.

## Claims

1. Apparatus for cleaning and sterilizing one or several mattresses,
wherein the apparatus comprises a mattress chamber having one compartment or several compartments each for accommodating one mattress, and wherein at least one supply air opening (2) and at least one exhaust air opening (3) are provided at the mattress chamber,
wherein the supply and exhaust air openings (2, 3) are arranged in the mattress chamber in such a manner that inflowing air or the like can stream in from the at least one supply air opening (2) through a mattress or through several mattresses and can stream out via the at least one exhaust air opening (3),
**characterized in that**, for cleaning, at least one brush (5) for cleaning the mattress surface is provided, in order to brush the mattress during the introduction into one compartment, and/or a device (6) for suctioning the mattress surface is provided, in order to suction the mattress during the introduction into the compartment, and
for sterilizing, a device (10) for ionizing the air, in particular dioxygen, is provided before the mattress chamber in the stream direction of the air, and, if necessary, a device for adding a disinfection agent into the air is provided before the mattress chamber in the stream direction of the air.

2. Apparatus according to claim 1, **characterized in that** press profiles are provided on the sides of the at least one compartment, in order to press on an introduced mattress laterally, wherein the press profiles are preferably formed so as to be adjustable.

3. Apparatus according to one of the previous claims, **characterized in that**, for each compartment, a perforated metal plate (4) is provided as a mattress support, wherein the perforated metal plate has, in particular, a homogeneous distribution of the perforations.

4. Apparatus according to one of the previous claims, **characterized in that** a device (9) for generating an irregular pulsed air stream is provided before the mattress chamber in the stream direction of the air.

5. Apparatus according to one of the previous claims, **characterized in that** a device for heating the air is provided before the mattress chamber in the stream direction of the air.

6. Apparatus according to one of the previous claims, **characterized in that** the air can be circulated in a circuit in the apparatus.

7. Apparatus according to claim 6, **characterized in that** a switching and/or control device for a stepped or progressive switching and/or controlling between air circulation in the apparatus and suctioning fresh air and blowing off used air is provided, wherein a combination of suctioning fresh air with blowing off used air, in particular, can also be adjusted.

8. Apparatus according to one of the previous claims, **characterized in that** a device for measuring the concentration of organic harmful substances is provided, wherein the measurement can be displayed, in particular, on a display device.

## Revendications

1. Appareil de nettoyage et de stérilisation d'un ou plusieurs matelas, dans lequel l'appareil comprend une chambre de matelas ayant un compartiment ou plusieurs compartiments destinés à accueillir chacun un matelas, et dans lequel au moins une ouverture d'arrivée d'air (2) et au moins une ouverture d'évacuation d'air (3) sont prévues dans la chambre de matelas, dans lequel les ouvertures d'arrivée et d'évacuation d'air (2, 3) sont disposées dans la chambre de matelas de telle manière que l'air entrant ou similaire puisse circuler depuis ladite au moins une ouverture d'arrivée d'air (2) à travers un matelas ou à travers plusieurs matelas et s'échapper par le biais de ladite au moins une ouverture d'évacuation d'air (3),
**caractérisé en ce que**, pour le nettoyage, au moins une brosse (5) destinée à nettoyer la surface d'un matelas est prévue afin de brosser le matelas lors de l'introduction dans un compartiment, et/ou un dispositif (6) destiné à aspirer la surface d'un matelas est prévu, afin d'aspirer le matelas lors de l'introduction dans le compartiment, et
pour la stérilisation, un dispositif (10) destiné à ioniser l'air, en particulier le dioxygène, est prévu avant la chambre de matelas dans la direction de circulation de l'air, et, si nécessaire, un dispositif d'addition d'un désinfectant dans l'air est prévu avant la chambre de matelas dans la direction de circulation de l'air.

2. Appareil selon la revendication 1, **caractérisé en ce que** des profils de pressage sont prévus sur les côtés dudit au moins un compartiment, afin de presser latéralement un matelas introduit, dans lequel les profils de pressage sont préférablement déplaçables.

3. Appareil selon l'une des revendications précédentes, **caractérisé en ce que**, pour chaque compartiment, une plaque de métal perforée (4) est prévue comme support de matelas, dans lequel ladite plaque de métal perforée comprend en particulier une distribution homogène des perforations.

4. Appareil selon l'une des revendications précédentes, **caractérisé en ce qu'**un dispositif (9) destiné à générer un flux d'air irrégulier à impulsions est prévu avant la chambre de matelas dans la direction de circulation de l'air.

5. Appareil selon l'une des revendications précédentes, **caractérisé en ce qu'**un dispositif destiné à chauffer l'air est prévu avant la chambre de matelas dans la direction de circulation de l'air.

6. Appareil selon l'une des revendications précédentes, **caractérisé en ce que** l'air peut être circulé dans un circuit dans l'appareil.

7. Appareil selon la revendication 6, **caractérisé en ce qu'**un dispositif de commutation et/ou commande destiné à une commutation et/ou une commande par étapes ou continue entre la circulation d'air dans l'appareil et l'aspiration d'air frais et l'évacuation d'air usagé est prévu, dans lequel une combinaison d'aspiration d'air frais avec l'évacuation d'air usagé peut aussi, en particulier, être ajustée.

8. Appareil selon l'une des revendications précédentes, **caractérisé en ce qu'**un dispositif de mesure de la concentration de substances toxiques organiques est prévu, dans lequel la mesure peut être, en particulier, affichée sur un dispositif d'affichage.
